# EUROPEAN PATENT APPLICATION

(11) **EP 1 170 002 A1**
(43) Date of publication of application: **09.01.2002**
(21) Application number: 00307057.0
(22) Date of filing: 17.08.2000
(51) Int. Cl.: A61K 7/48

(54) **Method of reducing the loss of skin elasticity and firmness**

(30) Priority: 29.06.2000 US 606889
(71) Applicant: JOHNSON & JOHNSON CONSUMER COMPANIES, INC., Skillman, NJ 08558 (US)
(72) Inventor: Shapiro, Stanley S., Livingston, NJ 07039 (US); Wiegand, Benjamin C., Newtown, PA 18940 (US); Martin, Katherine M., Ringoes, NJ 08551 (US)
(74) Representative: Fisher, Adrian John

(57) **Abstract**

The present invention relates to a method of reducing the loss of skin elasticity or firmness comprising topically administering a cosmetic composition incorporating a reducing amount of mineral water having a mineralization of at least about 200 mg/L into a cosmetic composition, wherein the mineral water comprises at least about 10 mg/L of calcium and at least about 5 mg/L of magnesium.

## Description

### FIELD OF THE INVENTION

The present invention relates to a method of reducing the loss or skin elasticity and firmness.

### BACKGROUND OF THE INVENTION

As human age, their skin loses its firmness and elasticity as a result of the breakdown of collagen and elastin in the skin. This skin aging process often resulting in the formation of wrinkles.

There are many cosmetic products on the market to help reduce the signs of skin aging. These products ma contain compounds such as retinoids and alpha hydroxy acids. These products, however, also often contain water. The application of water onto the skin actually reduces the firmness and elasticity of skin. Thus, the inclusion of water into cosmetic compositions, such as moisturizers, can actually reduce skin firmness and elasticity.

The present invention relates to the use of mineral water in cosmetic compositions to help reduce the loss of skin elasticity and firmness.

### SUMMARY OF THE INVENTION

The invention features a method of reducing the loss of skin elasticity comprising topically administering a cosmetic composition incorporating a reducing amount of mineral water having a mineralization of at least about 200 mg/L into a cosmetic composition, wherein the mineral water comprises at least about 10 mg/L of calcium and at least about 5 mg/L of magnesium. The present invention also features a method of reducing the ability of cosmetic composition to reduce the loss of skin elasticity or firmness, the method comprising incorporating a reducing amount of mineral water having a mineralization of at least about 200 mg/L into the cosmetic composition, wherein the mineral water comprises at least about 10 mg/L of calcium and at least about 5 mg/L of magnesium.

What is meant by reducing the loss of skin elasticity or firmness is either maintaining or increasing the firmness or elasticity of the skin (e.g., as measured by a cutometer). The loss of skin elasticity or firmness may be a result of a number of factors, including but not limited to aging, photo damage, or the result of an application of a cosmetic to the skin.

Other features and advantages of the present invention will be apparent from the detailed description of the invention and from the claims

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference.

The present invention relates to a method of using mineral water (e.g., a cosmetic composition comprising mineral water). What is meant by mineral water is water having mineralization (i.e., the sum of the concentrations of anions and cations present in the water) of at least about 200 mg/L (e.g., at least about 300 mg/L such from about 400 mg/L to about 1000 mg/L). Examples of such anions and cations include, but are not limited to, calcium, magnesium, bicarbonates, sulfates, potassium, sodium, chlorides, nitrates, phosphates, lithium, manganese, sulfites, fluoride, and iodide. In one embodiment, the mineral water has at least about 5 mg/L, e.g., at least about 10mg/L, of magnesium and at least about 10 mg/L, e.g., at least about 20 mg/L, of calcium.

The mineral water may be a naturally mineralized water, e.g., a mineral water suitable for consumption, or a thermal spring water, which is often not consumable. Examples of mineral water include, but are not limited to, eau d'Evian (Evian Eau Minerale Naturelle or Evian® Natural Spring Water referred herein as Evian® Mineral Water), eau Volvic, and eaux de Vittel (e.g., Grande Spring or Hepar Spring).

Examples of thermal spring waters include eau de la Bourboule, eau d'Enghien-les-bains, eau d'Allevard-les-bains, eau de Digne, eau des Maizieres, eau de Nyrac-les-bains, eau de Lons le Saunier, Eaux Bonnes, eau de Rochefort, eau de Saint Christau, eau des Fumades, eau de Tereau de Vittel, eaux du bassin de Vichy, eau d'Uriage, eau d'Avene, and eau de la Roche Posay.

In one embodiment, the mineral water comprises (a) from about 30 mg/L to about 150 mg/L of calcium; (b) from about 10 mg/L to about 50 mg/L of magnesium; (c) from about 150 mg/L to about 700 mg/L of bicarbonates; (d) from about 0.1 mg/L to about 5 mg/L of potassium; (e) from about 1 to about 20 mg/L of sulfates; (f) from about 1 to about 10 mg/L of sodium; (g) from about 1 mg/L to about 10 mg/L of chlorides; and (h) from about 1 mg/L to about 10 mg/L of nitrates.

In one embodiment, the mineral water is Evian® Mineral Water that comprises: (a) about 78 mg/L of calcium; (b) about 24 mg/L of magnesium; (c) about 357 mg/L of bicarbonates; (d) about 1 mg/L of potassium; (e) about 10 mg/L of sulfates; (f) about 5 mg/L of sodium; (g) about 4 mg/L of chlorides; and (h) from about 1 to about 4 mg/L nitrates.

What is meant by a reducing amount of mineral water is an amount capable of reducing the undesired effect (e.g., the reduction of the loss or elasticity and firmness in the skin of the user). In one embodiment, the composition comprises at least about 1%, by weight, of mineral water, e.g., about 1% to about 99%, by weight, of mineral water.

The methods of the present invention may further comprise topically administering (e.g., in a composition) one or more of the following compounds: creatine, carnitine, or pyruvic acid, or a cosmetically acceptable salt or ester thereof. What is meant by cosmetically acceptable salt or ester is one that does not eliminate the therapeutic benefit of the compound (e.g., its hydrating, nourishing, or metabolic enhancing properties). Examples of cosmetically acceptable salts, include, but are not limited to, those with cosmetically acceptable organic acids (e.g., acetic, lactic, maleic, citric, malic, ascorbic, succinic, benzoic, methesulfonic, toluenesulfonic, or pamoic acid), as well as polymeric acids (e.g., tannic or carboxymethyl cellulose) and salts with inorganic acids such as a hydrohalic acid (e.g., hydrochloric acid, sulfuric acid, or phosphoric acid). Examples of cosmetically acceptable esters include, but are not limited to, C2-C6 alkyl esters such as methyl esters and ethyl esters. Examples of such compounds include, but are not limited to, creatine monohydrate, creatine hemisulfate, D-carnitine, L-carnitine, L-carnitine hydrochloride, sodium pyruvate, and pyruvic acid methyl ester. As used herein, if the stereochemistry of the compound is not indicated, then the compound includes all stereoisomers, if any.

The compositions of the present invention may also comprise a nutrient, and emollient, and/or a humectant. What is meant by a nutrient is an organic substance occurring in foods that is not synthesized by the body and is necessary in trace amounts for the normal metabolic functioning of the body, such as vitamins, essential amino acids, and essential fatty acids.

Examples of such vitamins include, but are not limited to, vitamin A, a vitamin B (e.g., vitamin B1, vitamin B2, vitamin B6, or vitamin B12), vitamin C, and vitamin E (e.g., a tocopherol or a tocotrienol), and cosmetically acceptable salts and esters thereof, such a retinyl palmitate, retinyl acetate, tocopherol succinate, and tocopherol acetate.

Examples of such essential amino acids include, but are not limited to, arginine, histidine, isoleucine, leucine, lysine, methionine, phenylalanine, threonine, tryptophan, and valine.

Examples of essential fatty acids include, but are not limited to, linoleate and linolenate.

What is meant by an emollient is a compound that helps to maintain the soft, smooth, and pliable appearance of the skin (e.g., by remaining on the skin surface or in the stratum corneum to act as a lubricant). Examples of emollients can be found on pages 1657-1661 of the International Cosmetic Ingredient Dictionary and Handbook, eds. Wenninger and McEwen, pp. 1612-13, 1626, and 1654-55 (The Cosmetic, Toiletry, and Fragrance Assoc., Washington, D.C., 7^{th} Edition, 1997) (hereinafter "ICI Handbook"), and include, but are not limited to, hexyldecyl stearate and plant, nut, and vegetable oils such as macadamia nut oil, rice bran oil, grape seed oil, palm oil, primrose oil, hydrogenated peanut oil, olive oil and avocado oil.

What is meant by a humectant is a compound intended to increase the water content of the top layers of skin (e.g., hygroscopic compounds). Examples of humectants can be found on pages 1661-1662 of the ICI Handbook and include, but are not limited to, glycerin or trehalose (e.g., α,α- trehalose, β,β-trehalose, α,β-trehalose) or a salt or ester thereof (e.g., trehalose 6-phosphate).

The amount of carnitine or a cosmetically acceptable salt or ester thereof, creatine or a cosmetically acceptable salt or ester thereof, pyruvic acid or a cosmetically acceptable salt or ester thereof, nutrient, emollient, or humectant in the composition varies (e.g., depending on the intended use or the form of the composition) being administered and will typically be present in the composition in an amount from about 0.001% to about 20%, by weight, of the topically applied composition, e.g., from about 0.01% to about 10%, by weight, such as from about 0.01% to about 5%, by weight, of such emollient or humectant and from about 0.001% to about 10%, by weight, of the topically applied composition, e.g., from about 0.01% to about 5%, by weight, such as from about 0.01% to about 1%, by weight, of such carnitine, creatine, pyruvic acid or cosmetically acceptable salt or ester thereof.

In one embodiment, the method further comprises administering (e.g., in a composition) another cosmetically active agent. What is meant by a "cosmetically active agent" is a compound that has a cosmetic or therapeutic effect on the skin, e.g., agents to treat wrinkles, acne, or to lighten the skin. In one embodiment, the agent is selected from, but not limited to, the group consisting of hydroxy acids, benzoyl peroxide, sulfur resorcinol, ascorbic acid, D-panthenol, hydroquinone, sunscreen agents, anti-inflammatory agents, skin lightening agents, antimicrobial and antifungal agents, estrogens, 2-dimethylaminoethanol, lipoic acid, amino acids such a proline and tyrosine, lactobionic acid, acetyl-coenzyme A, niacin, riboflavin, thiamin, ribose, electron transporters such as NADH and FADH2, botanical extracts such as aloe vera and soy, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of hydroxy acids include, but are not limited, to (i) alpha-hydroxy acids such as glycolic acid, lactic acid, malic acid, citric acid, and tartaric acid, (ii) beta-hydroxy acids such as salicylic acid, and/or (iii) polyhydroxy acids. See, e.g., European Patent Application No. 273,202.

Examples of derivatives of ascorbic acid include, but are not limited to, ascorbyl palmitate, magnesium ascorbyl phosphate, sodium ascorbyl phosphate, zinc ascorbyl phosphate, ascorbyl glucoside, sodium ascorbate, and ascorbyl polypeptide. An example of a derivative of hydroquinone includes, but is not limited to, arbutin.

The method of the present invention can be practiced by topically administering to a mammal, e.g., by the direct laying on or spreading on the skin of a human, the mineral water and any other ingredients in a composition. The compositions (e.g., cosmetic compositions) useful in the subject invention involve formulations suitable for topical application to mammalian skin, a formulation comprising (i) mineral water, (ii) optionally, a safe and effective amount of creatine, carnitine, or pyruvic acid, or a cosmetically acceptable salt or ester thereof, (iii) optionally a nutrient, an emollient, humectant (e.g., trehalose), or other cosmetically active agent(s), and (iv) optionally, a cosmetically-acceptable topical carrier. The term "cosmetically-acceptable topical carrier" refers to a carrier for topical use that is capable of having the mineral water and any other agents dispersed or dissolved therein, and possessing acceptable safety properties. In one embodiment, the cosmetically-acceptable carrier comprises mineral water (e.g., an emulsion where the aqueous phase comprises mineral water or a mineral water based cleanser or spray).

The topical compositions useful in the present invention may be used for a variety of cosmetic uses, including, but not limited to, treating, cleansing, beautifying, or covering the skin or hair of a human). The compositions, thus, may be made into a wide variety of product types. These include, but are not limited to lotions, creams, gels, sticks, sprays, ointments, pastes, mousses, shampoos, cosmetics, and dermal patches. Products include, but are not limited to, lip balms, moisturizing and sunscreen lotions/creams, skin cleansing compositions (e.g., facial scrubs), and body mists. These products may comprise several types of carrier systems including, but not limited to single phase solutions (e.g., water, such as mineral water, or oil based solutions), emulsions, and gels.

The topical compositions useful in the present invention formulated as solutions typically include a cosmetically acceptable water, mineral water, and/or organic carriers (e.g., from about 80% to about 99.99%, by weight, of the composition, such as from about 90% to about 99%, by weight of the composition, of an acceptable water, mineral water, or organic solvent). Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, isopropanol, butanetriol, sorbitol esters, 1,2,6-hexanetriol, butanediol, and mixtures thereof.

If the topical solution useful in the present invention are formulated as an aerosol and applied to the skin as a spray-on, a propellant is added to a solution composition. Examples of propellants useful herein include, but are not limited to, chlorinated, fluorinated, and chloro-fluorinated lower molecular weight hydrocarbons. Other propellants useful herein can be found in Sagafin, Cosmetics Science and Technology, 2nd Edition, Vol. 2, pp. 443-65 (1972) (hereinafter "Sagafin") and the ICI Handbook pp. 1655.

A lotion can be made from a solution carrier system. Lotions typically comprise from about 1% to about 20% by weight of the composition (e.g., from about 5% to about 10%) of an emollient(s) and from about 50% to about 90% by weight of the composition (e.g., from about 60% to about 80%) of water, e.g., mineral water.

Another type of product that may be formulated from a solution carrier system is a cream. A cream typically comprises from about 5% to about 50% by weight of the composition (e.g., from about 10% to about 20%) of an emollient(s) and from about 45% to about 85% by weight of the composition (e.g., from about 50% to about 75%) of water.

Yet another type of product that may be formulated from a solution carrier system is an ointment. An ointment may comprise a simple base of animal or vegetable oils or semi-solid hydrocarbons. Ointments may also comprise absorption ointment bases that absorb water to form emulsions. Ointment carriers may also be water-soluble. An ointment may comprise from about 1% to about 20% by weight of the composition of an emollient(s) plus from about 0.1% to about 2% by weight of the composition of a thickening agent(s). A more complete disclosure of thickening agents or viscosity increasing agents useful herein can be found in Sagafin pp. 72-73 and the ICI Handbook pp. 1693-97.

If the carrier is formulated as an emulsion (e.g., an oil-in-water, silicone-in-water, water-in-oil, or water-in-silicone emulsion), from about 1% to about 10% by weight of the composition (e.g., from about 2% to about 5%) of the carrier system may comprise an emulsifier(s). Emulsifiers may be nonionic, anionic, cationic, or zwitterionic. Suitable emulsifiers are disclosed in, for example, U.S. Patent No. 3,755,560, U.S. Patent No. 4,421,769, McCutcheon's Detergents and Emulsifiers, North American Edition, pp. 317-24 (1986), and the ICI Handbook, pp.1673-86.

Lotions and creams can also be formulated as emulsions. Typically, such emulsions may comprise from 0.5% to about 5% by weight of the composition of an emulsifier(s). Creams may typically comprise from about 1% to about 20% by weight of the composition (e.g., from about 5% to about 10%) of an emollient (s); from about 20% to about 80% by weight of the composition (e.g., from 30% to about 70%) of water such as mineral water; and from about 1% to about 10% by weight of the composition (e.g., from about 2% to about 5%) of an emulsifier(s).

Two phase emulsion skin care preparations, such as lotions and creams, of the oil-in-water type and water-in-oil type are well-known in the cosmetic art and are useful in the subject invention. Triphase emulsion compositions, such as the water-in-oil-in-water type, as disclosed in U.S. Patent No. 4,254,105, are also useful in the subject invention. In general, such triphase emulsions contain water, emollients, and emulsifiers as essential ingredients. Triple emulsion carrier systems comprising an oil-in-water-in-silicone fluid emulsion composition, as disclosed in U.S. Patent No. 4,960,764, may also be useful in the subject invention.

The methods of the present invention may also comprise administering a composition containing one or more of the following: antioxidants (e.g., ascorbic acid, tocopherols, BHA, polyphenols, tocotrienols, and BHT), chelating agents (e.g., EDTA), and preservatives (e.g., parabens). Examples of suitable antioxidants, preservatives, and chelating agents are listed in pp. 1612-13, 1626, and 1654-55 of the ICI Handbook. In addition, the topical compositions useful herein can contain conventional cosmetic adjuvants, such as dyes, opacifiers (e.g., titanium dioxide), pigments, and fragrances.

The compositions of the present invention may be prepared using methodology that is well known by an artisan of ordinary skill. The following is a description of the testing and manufacturing of cosmetic compositions of the present invention.

### Example 1: Mineral Water Containing Carnitine, Trehalose, and Sodium Pyruvate

A composition containing Evian® Mineral Water (Evian, France), carnitine, sodium pyruvate, and trehalose, was manufactured using the ingredients listed in Table I.

**TABLE I**

| **INGREDIENTS** | **% Weight** |
|---|---|
| L-Carnitine | 1 |
| Sodium Pyruvate | 1 |
| Trehalose | 1 |
| D-Panthenol (75%)/ Water (25%) | 1.3 |
| Magnesium Ascorbyl Phosphate | 1 |
| L-Proline | 1 |
| Mineral Water | 72.7 |
| Pentylene Glycol | 20 |
| Phenoxyethanol | 1 |

The mineral water was first heated to 30°C. The other ingredients were then added and dissolved one by one under mixing conditions. The pentylene glycol was obtained from Dragoco Gerberding & Co. (Holzminden, Germany) under the trade name Hydrolite®-5.

### Example 2- Sunscreen Moisturizer

A sunscreen moisturizing composition containing the composition of Example 1 and the sunscreen octyl methoxycinnamate is manufactured using the ingredients listed in Table II.

**Table II**

| **INGREDIENTS** | **% Weight** |
|---|---|
| **Water Phase Ingredients** | |
| Mineral Water | q.s. 100 |
| Disodium EDTA | 0.1 |
| Glycerin | 3 |
| Butylene glycol | 3 |
| Carbomer | 0.25 |
| Acrylate-C10-30 alkyl acrylate crosspolymer | 0.07 |
| Glyceryl polymethacrylate 67% / water 32% / propylene glycol 1% | 5 |
| Propyl paraben | 0.201 |
| Methyl paraben | 0.35 |
| Phenoxyethanol | 0.584 |

| **Oil Phase Ingredients** | |
|---|---|
| Cetearyl alcohol | 1 |
| C12-C15 alkyl benzoate | 4 |
| Potassium cetyl phosphate | 1.5 |
| PEG-100 stearate 50%/ glyceryl stearate 50% | 0.3 |
| Di-C12-13 alkyl malate | 5 |
| Talc | 1 |
| Phenoxyethanol | 0.365 |
| Propyl paraben | |
| Methyl paraben | |
| Iodopropynyl butylcarbamate 10%/ PEG-4 laurate 90% | 0.1 |
| Octyl methoxycinnamate | 7.5 |
| Butyl methoxydibenzoylmethane | 3 |
| Tocopheryl acetate | 1 |

| **Tromethamine Mixture** | |
|---|---|
| Mineral Water | 2 |
| Tromethamine | 0.3 |

| **Trehalose Mixture** | |
|---|---|
| Mineral Water | 2 |
| Trehalose | 0.25 |

| **Post Addition Ingredients** | |
|---|---|
| Cyclomethicone | 2 |
| Composition of Example 1 | 1 |
| Evening primrose oil | 0.01 |
| Fragrance | 0.3 |

To form the water phase, the mineral water (Evian® Mineral Water, Evian, France) of the Water Phase Ingredients (Evian® Mineral Water) was heated to 85°C and stirred for about 15 minutes in a first beaker. The disodium EDTA, glycerin, and butylene glycol were then added to the first beaker and stirred for an additional 10 minutes. The first beaker was then cooled to 82°C. Next, the carbomer and the acylate-C10-30 alkyl acrylate crosspolymer were dispersed in the mixture in the first beaker and stirred for about 25 minutes until the mixture gelified. The remaining Water Phase Ingredients were then added to the first beaker and mixed.

To form the oil phase, the Oil Phase Ingredients were added to a second beaker, heated to 85°C, and mixed for 15 minutes. The oil phase mixture in the second beaker was then added to the first beaker under mixing conditions to form an emulsion. The emulsion was then cooled to 25°C and neutralized with the Tromethamine Mixture. Next, the cyclomethicone was mixed into the emulsion for 15 minutes. Lastly, the Trehalose Mixture, the composition of Example 1, the evening primrose oil, and the fragrance were added to the resulting mixture and mixed until uniform.

### Example 3 : Promotion of Skin Elasticity

A study was performed on 14 human panelists to measure the effect of skin elasticity/firmness with both distilled water and mineral water (Evian® Mineral Water). Elasticity measurements were made using a Cutometer (Model No. SEM-575, Courage & Khazaka, Electronic GmbH, Koeln, Germany). See Handbook of Non-Invasive Methods and the Skin, eds. J. Serup & G. Jemec, Chapter 14.3 (1995). The two products were rotated on the different forearms of the panelists, so that there was an even distribution of products on each forearm during the study. The protocol for the study is recited below.

Baseline cutometer measurements were taken before the forearms were soaked. Each volar forearm was soaked in a water solution for five minutes, either with deionized water or with the mineral water. The forearm was then removed from the water bath and was blotted dry. After drying for a period of a approximately five minutes, cutometer measurements were taken to measure the elasticity levels in the skin. The same was repeated for the other volar forearm.

Mineral water was surprisingly found to preserve the skin's elasticity whereas distilled water was found to reduce skin elasticity by ten percent. These results indicate that skin care compositions manufactured with mineral water will better at promoting skin elasticity and firmness than a composition containing only distilled water.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A cosmetic method of reducing the loss of skin elasticity, said method comprising topically administering a cosmetic composition incorporating a reducing amount of mineral water having a mineralization of at least about 200 mg/L into a cosmetic composition, wherein said mineral water comprises at least about 10 mg/L of calcium and at least about 5 mg/L of magnesium.

2. A cosmetic method of promoting the ability of cosmetic composition to reduce the loss of skin elasticity, said method comprising incorporating a reducing amount of mineral water having a mineralization of at least about 200 mg/L into said cosmetic composition, wherein said mineral water comprises at least about 10 mg/L of calcium and at least about 5 mg/L of magnesium.

3. A cosmetic method of reducing the loss of skin firmness, said method comprising topically administering a cosmetic composition incorporating a reducing amount of mineral water having a mineralization of at least about 200 mg/L into a cosmetic composition, wherein said mineral water comprises at least about 10 mg/L of calcium and at least about 5 mg/L of magnesium.

4. A cosmetic method of promoting the ability of a cosmetic composition to reduce the loss of skin firmness, said method comprising incorporating a reducing amount of mineral water having a mineralization of at least about 200 mg/L into said cosmetic composition, wherein said mineral water comprises at least about 10 mg/L of calcium and at least about 5 mg/L of magnesium.

5. A method of any preceding claim, wherein said mineral water comprises (a) from about 30 mg/L to about 150 mg/L of calcium; (b) from about 10 mg/L to about 50 mg/L of magnesium; (c) from about 150 mg/L to about 700 mg/L of bicarbonates; and (d) from about 0.1 mg/L to about 5 mg/L of potassium.

6. A method of claim 5, wherein said mineral water further comprises (e) from about 1 to about 20 mg/L of sulphates; (f) from about 1 to about 10 mg/L of sodium; (g) from about 1 mg/L to about 10 mg/L of chlorides; and (h) from about 1 mg/L to about 10 mg/L of nitrates.

7. A method of any preceding claim, wherein said mineral water is Evian® Mineral Water.

8. A method of any preceding claim, wherein said method further comprises administering a compound selected from the group consisting of creatine, carnitine, and pyruvic acid, and a cosmetically acceptable salt or ester thereof.

9. A mineral water as recited in any of claims 1, 5, 6 or 7, for use in the therapeutic treatment of skin to reduce the loss of skin elasticity or firmness.

10. The use of a mineral water as recited in any of claims 1, 5, 6 or 7 in the manufacture of a medicament for the therapeutic treatment of skin to reduce the loss of skin elasticity or firmness.
